# EUROPEAN PATENT APPLICATION

(11) **EP 4 636 779 A1**
(43) Date of publication of application: **22.10.2025**
(21) Application number: 24170483.2
(22) Date of filing: 16.04.2024
(51) Int. Cl.: G16H 40/60, G16H 40/63, G16H 40/67

(54) **REMOTE-SAFE IMAGING SYSTEM**

(71) Applicant: Koninklijke Philips N.V., 5656 AG Eindhoven (NL)
(72) Inventor: AMTHOR, Thomas Erik, Eindhoven (NL); VAN ECK, Menno, Eindhoven (NL); VAN DEN BRINK, Johan Samuel, 5656AG Eindhoven (NL)
(74) Representative: Philips Intellectual Property & Standards

(57) **Abstract**

Methods and systems for operating a medical system are presented. The method (400) comprises: receiving (402) commands from local and/or remote universal serial bus, USB, input devices through a USB interface, wherein each of the local and remote USB input devices (202, 204) has unique USB input device identifier; comparing (404) USB input device identifiers with predetermined local USB input device identifiers, thereby identifying whether the commands originate from local or remote USB devices; determining (406) an action to be pursued for the received commands based on where the commands originate from; selectively transmitting (408) commands to the medical system based on the determined actions. The system comprises a computational system configured to perform the method.

## Description

### TECHNICAL FIELD

The subject matter described herein relates to imaging systems, in particular to remote operation of the imaging systems.

### BACKGROUND

Remote assistance for diagnostic imaging has gained popularity in recent years. In most hospital settings, imaging systems of more than one vendor need to be connected to a remote assistance solution, so that solutions provide multi-vendor connectivity. A typical solution for remotely connecting to a medical device without the need for any additional software on that device is to transmit mouse and keyboard commands from the remote operating station to the medical device and inject these commands through the Universal Serial Bus (USB) port of the devices.

The information included in this Background section of the specification, including any references cited herein and any description or discussion thereof, is included for technical reference purposes only and is not to be regarded as subject matter by which the scope of the disclosure is to be bound.

### SUMMARY

The objective of the invention is to differentiate whether an imaging system is operated remotely. A further object of the invention is to selectively prevent safety-critical actions in remote operation of the system. A yet further object of the invention is to ensure quality control of remote-operated imaging systems.

In a first aspect of the invention a method of operating a medical system is provided, comprising:
receiving commands from local and/or remote USB input devices through a USB interface, wherein each of the local and remote USB input devices has an associated unique USB input device identifier;
comparing USB input device identifiers with predetermined local USB input device identifiers, thereby identifying whether the commands originate from local or remote USB devices;
determining an action to be pursued for the received commands based on where the commands originate from;
selectively transmitting commands to the medical system based on the determined actions.

In some of the embodiments a locally stored configuration list comprises the predetermined local USB identifiers. In some embodiments the method may comprise: requesting a user to interact with a local USB input device; assigning the local USB input device a local USB input device identifier; storing the local USB input device identifier as configuration list in a memory. In some embodiments the method may comprise permitting a user to modify the predetermined local USB device identifiers by editing the configuration list. The user in this context may be any of a local user, a remote user, an authorized representative of a medical device manufacturer and an authorized entity.

In some embodiments determining the action to be pursued for the received commands is further based on authorization level associated to the USB input device identifier. The authorization level may be comprised in an authorization configuration list stored in a memory and wherein the authorization configuration list is editable for assigning authorization level to remote USB input device identifiers.

Selectively transmitting commands to the medical system may comprise blocking at least a command received from the remote USB input device. The medical system may be understood as a console or operating system associated to single or multiple medical systems, such as for example medical imaging systems.

In some of the embodiments the method may comprise indicating that commands of a remote USB input device are selectively transmitted to the medical system. In some of the embodiments indicating comprises alerting a local user for attempted unauthorized commands from a remote USB input device. Additionally or alternatively, indicating may comprise remote monitoring of attempted unauthorized commands from a remote USB input device.

In some of the embodiments the method may further comprise logging the action for the received commands originating from remote USB input devices. Logging may comprise log-entries of at least one of a timestamp, user action, examination context.

In another aspect the invention provides a system for operating a medical system, comprising:
a USB interface adapted for connection of local and remote USB input devices;
a computational system configured to perform any of the hereinbefore disclosed method embodiments. The medical system may be a medical imaging system, such as for example a magnetic resonance imaging (MRI) system, a computer tomography (CT) system, a diagnostic X-ray (DXR) system, radiation therapy (MR-Linac) system. Other examples of medical systems may be any of radiotherapy treatment planning systems, radiology reading, reporting tools, patient monitoring devices, ventilators, injectors.

In yet a further aspect of the invention a computer program is presented, comprising machine executable instructions, wherein execution of the machine executable instructions causes a computational system to perform any of the method embodiments discussed hereinbefore.

Features described above with respect to method embodiments are applicable and are contemplated also for corresponding system embodiments, with similar benefits.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the system, as defined in the claims, is provided in the following written description of various embodiments of the disclosure and illustrated in the accompanying drawings.

### BRIEF DESCRIPTION OF THE DRAWINGS

Illustrative embodiments of the present disclosure will be described with reference to the accompanying drawings, of which:
Figure 1 illustrates an example of a system for operating a medical system;
Figure 2 illustrates schematically an embodiment according to the invention;
Figure 3 illustrates an example of a USB device identifier in a Windows operating system;
Figure 4 illustrates schematically a method of operating a medical system according to the invention.
Figure 5 illustrates data flow for an exemplary embodiment according to the invention.

### DETAILED DESCRIPTION

For the purposes of promoting and understanding the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately. Features described in relation to a system, may be implemented in a computer implemented method and/or in a computer program product, in a corresponding manner.

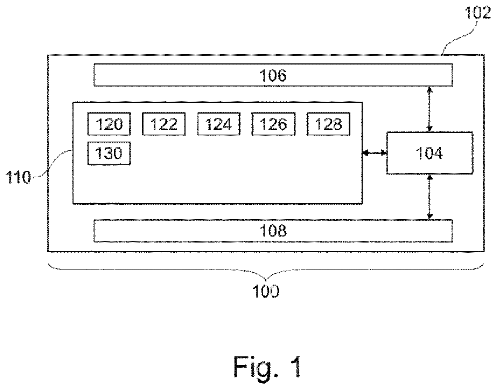

Fig. 1 illustrates an example of a system 100 for operating a medical system. The system 100 may be termed a control system or console. In some embodiments the system 100 may be part of a medical system. In any of the embodiments the medical system may be a medical imaging system, such as for example one of the MRI, CT, DXR, MR-Linac systems or their combination. Further medical systems benefiting from the invention may be any of radiotherapy treatment planning systems, radiology reading, reporting tools, patient monitoring devices, ventilators, injectors, of which settings parameters may be remotely controlled.

In this example, the system comprises a computer 102. The computer 102 may represent one or more computer systems that are in one location or are distributed. The computer 102 is shown as including a computational system 104. The computational system 104 may for example be one or more processing cores that are located at one or more locations. The computational system 104 is shown as being connected to an optional hardware interface 106. The hardware interface 106 may for example be used to connect the computational system 104 with other components of one or more medical systems 100 with provenience from single or multiple vendors, if they are present. The hardware interface 106 may enable the computational system 104 to control such components, like for example disclosed in WO 2022/013023 A1, which is hereby incorporated by reference in its entirety, in which the hardware interface is connected to a transceiver and a gradient controller of an MRI system.

The computer 102 is further shown as comprising a user interface 108. The user interface 108 may for example be used by an operator to control the operation and function of the system 100 either locally or remotely. The system 100 could be a standalone computer system but it could also be integrated into one of the one or more medical systems.

The computer system is further shown as comprising a memory 110. The memory 110 is intended to represent any combination of memory or storage device which is accessible to the computational system 104. The memory 110 may include volatile and nonvolatile memory storage means and components. The memory 110 in some embodiments may be based on or may rely on cloud-based data stored in logical pools across disparate, commodity storage servers located on premises or in a data center managed by a third-party cloud provider. Accordingly, the memory as schematically depicted in Fig. 1 is just for illustration purposes. Therefore, any or all components comprised in memory 110 may be cloud-based.

The memory 110 is shown as containing machine-executable instructions 120. The machine-executable instructions 120 may be configured to enable the computational system 104 to perform basic data processing and image processing tasks, such as for example reconstructing medical images. The machine-executable instructions 120 may also in some examples contain code which enables the computational system to control other components via the optional hardware interface 106.

The memory 110 may further comprise image segmentation algorithm 122. The image segmentation algorithm 122 may be configured for outputting one or more predetermined anatomical regions for medical imaging data. This may be for a particular field of view or anatomical region of the subject, e.g., one or multiple anatomical structures or organs of a patient.

The memory 110 may comprise or store the measured medical imaging data 124. The memory 110 may further comprise an image segmentation 126 that has been received from the image segmentation algorithm 122 by inputting the measured medical imaging data 124. The memory 110 may comprise a selected image portion 128. This is one or more regions or anatomical regions that have been identified in the image segmentation 126. The selected image portion 128 may for example be selected using a predetermined criterion that is applied to the image segmentation 126. The memory may comprise one or a plurality of various algorithms 130 for deriving parameters within a region of interest of an imaging zone based on the medical imaging data 124 and by optionally using any information present in the memory 110 and/or information that may be input by a user through the user interface 108.

The memory 110 may further comprise operating commands of the medical system, for example to acquire the medical imaging data. In an example, the operating command comprise pulse sequence commands for an MRI system.

In most hospital settings, imaging systems of more than one vendor need to be connected to a remote assistance solution, so that the solution can provide multi-vendor connectivity. Such solution may be termed Operations Command Center (OCC), an example may be radiology OCC (ROCC).

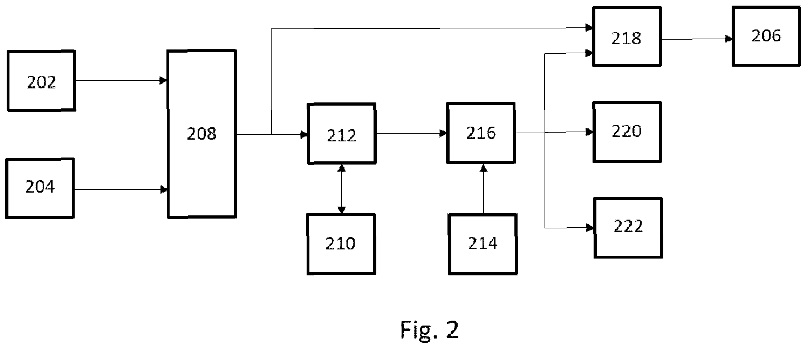

With reference to Fig. 2, a schematic diagram illustrates embodiments of the invention, which are capable of discriminating between local and remote USB devices, may detect remote commands and determine their safety implications, and/or act upon remote commands via selective blocking, user interface (UI) notification, logging, by using a decision logic. In some of the embodiments one or more elements may be missing, depending on the objective to be realized by the embodiment.

The Universal Serial Bus interface 208 may be comprised by or be the user interface 108 presented with reference to Fig. 1. Local USB devices illustrated with 202 and/or remote USB input devices illustrated with 204 are connectable to the USB interface 208. The functionalities illustrated with reference to Fig. 2 may be performed by a processor of the computer 102 or the computational system 104.

In a typical remote access setting, remote access is provided through the means of keyboard, video, mouse (KVM) switch that provides USB human interface device (HID) commands similar to the locally connected mouse and keyboard. Current imaging systems can, therefore, typically not distinguish between remote and local operation.

Connecting remotely to an imaging system can have consequences regarding safety associated to patient and/or the system, data privacy and security, and liability. Therefore, it is desirable that a medical system, e.g., an MRI, CT etc., can automatically detect if it is operated remotely, to selectively prohibit safety-critical actions and/or to record remote actions for quality control.

From a liability perspective, it is further desirable for a medical system vendor to be able to differentiate and in case it is required, to provide evidence that a system fault or safety-critical event has been initiated by a remote action of a different party or by intended local operation.

The invention intends to address at least some of these items by various embodiments.

Local USB input devices 202, such as mouse and/or keyboard on the local operator's desk, and remote USB input devices 204, such as mouse and/or keyboard transmitted via a KVM device, are connected to the USB interface 208 of the console or computer 102. The USB input devices 202 and 204 may alternatively or additionally be any other USB HID, such as touchpad, trackpad, graphics tablet, joystick.

The USB commands received via the USB interface are analyzed by the input identification module 212. The input identification module compares the USB input device identifiers transmitted alongside the USB command with a list of known USB input device identifiers to determine if the USB command originates from a local USB input device or a remote USB input device. The list of known USB input device identifiers may comprise identifiers of solely local USB input devices or a combination of identifiers of local and remote USB input devices.

The input configurator 210 provides information about local USB input device identifiers to the identification module. It can be implemented as a file storing the USB device identifiers or as a user interface allowing a user to specify which USB device is a local input device.

In one embodiment of a UI-driven input configurator, a user may be asked to move the local mouse and/or press a key on the local keyboard, while the input configurator receives the USB identifiers of the respective USB commands from the input identification module and stores them in the configuration settings. With other words, the system registers the USB input device identifiers of the devices sending mouse and keyboard commands at that point in time. These device identifiers are further regarded to be the local USB input devices for discriminating them from any of the remotely connected USB input devices.

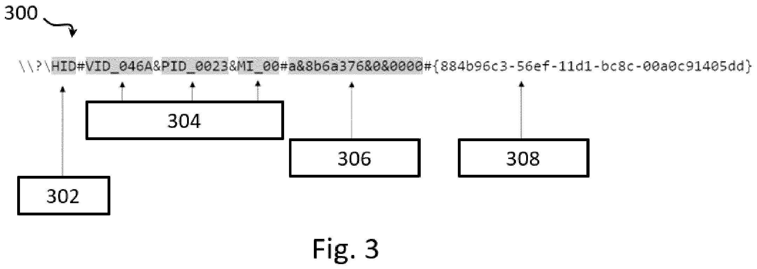

Fig. 3 shows an example of a USB input device identifier code 300 as generated by the Windows operating system. For each local input device, such a device identifier can be stored by the input configurator, wherein 302 relates to the device type such as HID for USB devices, Advanced Configuration and Power Interface (ACPI) for built-in laptop keys/touchpad; 304 relates to device vendor identifier, product identifier and revision number; 306 relates to physical connection identifier, such as USB port, etc.; 308 relates to the globally unique identifier (GUID), assigned by device driver.

Further referring to Fig. 2, after identification of a local or remote USB command, the origin and type of the command are passed on to the decision logic 216. The purpose of the decision logic is to determine which action to take for each received USB command, depending on its origin and type. The decision logic may control three additional modules: the selective USB command blocker 218, the remote access action visualization module 220, and the remote access action logging module 222. In some embodiments at least one of the remote access action visualization module 220 and the remote access action logging module 222 might not be present.

The decision logic 216 receives context information from the console status and authorization configuration module 214. This context information includes information about the authorization level of the remote user, such as for example the remote user may not open patient database and/or may not start a procedure, e.g., start a scanning procedure, but it is authorized to modify settings parameters, e.g., scan settings parameters. The context information may further comprise information about the status of the console UI, such as for example current mouse cursor position, position and status of UI elements on the console screen, etc.

The selective USB command blocker 218 is controlled by the decision logic 216. Depending on the control input to the decision logic 216, it either passes a USB command on to the console operating system to be processed 206 as usual, or blocks an incoming USB command, i.e. does not pass it on to further processing.

The remote access action visualization module 220 may also be controlled by the decision logic 216. Depending on the control input to the decision logic 216, it may visualize a graphical element on the console user interface, such as a red screen border, an indicator at the mouse cursor, and/or an alert message.

The remote access action logging module 222 may also be controlled by the decision logic 216. Depending on the control input to the decision logic 216, it may record any of the time, type, and origin of a USB command and the current UI status. In this way, the records can be used to determine what UI action was caused by the respective USB commands.

During medical procedure, e.g., diagnostic imaging examination, image guided therapy, the control input to the decision logic 216 are the input identification, the console status, authorization configuration, and depending on those control input, the decision logic decides how to proceed for each received USB command and controls the output modules 218, 220, 220, accordingly. A typical nonlimiting implementation example may be as follows: in case that a local USB command is received, the decision logic instructs the selective USB command blocker 218 to pass the command on to the console as usual and not to initiate any visualization and/or logging action.

In case that a remote USB mouse click is received, the decision logic checks the current mouse cursor position from the console context. If the mouse cursor is positioned on a UI element that the remote user is not authorized to click, it will instruct the selective USB command blocker 218 to block this click event. It may further instruct the visualization module to display an alert message for the local user. It may further instruct the logging module to record the click event together with the information that the mouse click was intended to be executed on a certain UI element, but was blocked.

In case that a remote USB action is received that the remote user is authorized to perform, the command is passed on to the console processing 206, but the action may still be recorded by the USB action logger to serve as evidence of remote user action in case of a security or safety audit. The remote access action visualization 220 may be configured to, when remote interaction with the system is detected, provide UI alerts to the local user, wherein nonlimiting examples may comprise: while the remote user is actively moving the mouse cursor or is typing, the remote action can be indicated by a red border around the screen; and/or while the remote user is actively moving the mouse cursor or is typing, the remote action can be indicated by a modified version of the mouse or text curser, e.g., a larger colorful version of the normal cursor.

Nonlimiting examples of remote access action logging 222 may comprise log-entries such as timestamp, (remote) user action, exam context, etc., exemplarily listed in Table 1.

**Table 1**

| **Timestamp** | **Device ID** | **Device type** | **Action** | **Patient context** | **Exam context** |
|---|---|---|---|---|---|
| 2024-03-28 10:02:32 | 2db270-aa3fc8 | mouse | Click on "STOP SCAN" button | Patient ID 147328 | T1W scan, progress 65% |
| 2024-03-28 11:19:05 | 8b62d1-4ce932 | keyboard | Pressed F4 key | Patient ID 218437 | Exam finished, patient still in bore |

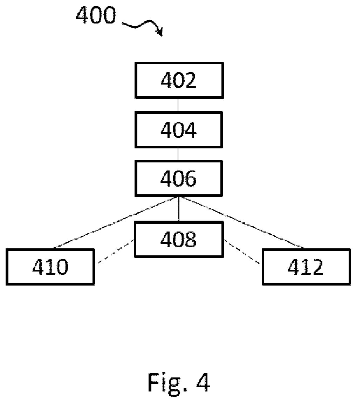

In yet another aspect of the invention a method 400 of operating a medical system, illustrated schematically in Fig. 4, is disclosed, comprising:
receiving 402 commands from local and remote USB input devices 202, 204 through a USB interface 208, wherein each of the local and remote USB input devices has unique USB input device identifier;
comparing 404 USB input device identifiers with predetermined local USB device identifiers, thereby identifying whether the commands originate from local or remote USB input devices;
determining 406 an action to be pursued for the received commands based on where the commands originate from;
selectively transmitting 408 commands to the medical system based on the determined actions.

In some of the embodiments the method may further comprise: requesting a user to interact with a local USB input device; assigning the local USB input device a local USB input device identifier; storing the local USB input device identifier as configuration list in a memory. Additionally or alternatively, the method may comprise allowing the user to modify the predetermined local USB device identifiers by editing a configuration list. In some embodiments, additionally or alternatively, the user may be a local end-user or a remote user, such as an authorized representative of the manufacturer of the medical device 206. In some of the embodiments the authorized representative of the manufacturer and/or an authorized entity is allowed to adjust the configuration list of the authorized USB input device identifiers. In some of the embodiments only the authorized representative of the manufacturer and/or authorized entity is allowed to adjust the configuration list of the authorized USB input device identifiers.

In any of the embodiments determining 406 the action to be pursued for the received commands may further be based on authorization level associated to the USB input device identifier, e.g., remote user may not be permitted to open patient database and/or to start a scanning procedure, but it may be authorized to modify scan settings parameters. Additionally or alternatively, determining 406 the action to be pursued for the received commands may be based on context information, which may include status of the console UI, e.g., current mouse cursor position, position and status of UI elements on the console screen, etc.

In some of the embodiments the authorization level is comprised in an authorization configuration list stored in a memory and wherein the authorization configuration list is editable for assigning authorization level to remote USB input device identifiers.

In some of the embodiments selectively transmitting commands to the medical system may comprise blocking at least a command received from the remote USB input device.

In some of the embodiments the method may further comprise indicating 410 that commands of a remote USB input device are selectively transmitted to the medical system. In some of the embodiments this may be in the form of alerting a local user or end-user for attempted unauthorized commands from a remote USB input device, for example by presenting a graphical element on the console user interface, such as a red screen border on a display, an indicator at the mouse cursor, and/or an alert message.

Additionally or alternatively, indicating may comprise remote monitoring of attempted unauthorized commands from a remote USB input device. In an example the remote monitoring comprises alerting an authorized representative of the medical device manufacturer and/or an authorized entity for attempted unauthorized commands from a remote USB input device.

In some of the embodiments the method may further comprise logging 412 the action for the received commands originating from remote USB input device. Logging may comprise log-entries of at least one of a timestamp, user action, examination context.

In some of the embodiments when a command is received from local USB input device, the command is passed on to the medical system, e.g., console, control computer of the medical system, without initiating any visualization or logging action.

In some of the embodiments logging 412 the action and indication 410 of command originating from remote USB input device may be upon determining 406 an action to be pursued for the received commands based on where the commands originate from, and in some embodiments it may be triggered only by selectively transmitting 408 the command to the medical system, indicated with dashed lines in Figure 4.

Additionally or alternatively, logging 412 may comprise remote logging of attempted unauthorized commands from a remote USB input device, that is accessible by an authorized representative of the medical device manufacturer and/or authorized entity, for example to provide evidence of remote user action in the event of a security or safety audit.

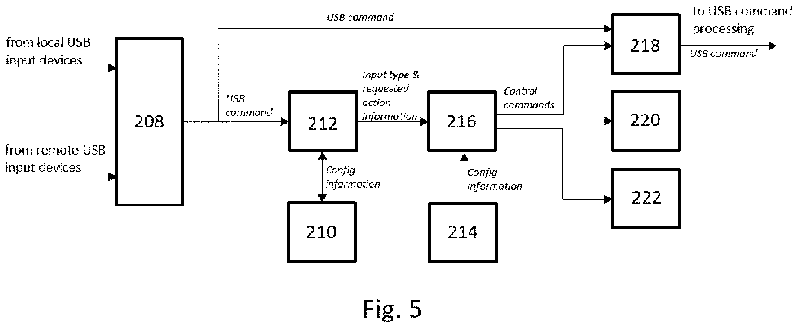

With reference to Fig. 5 an embodiment is illustrated comprising exemplary data flow, including an indication of the type of data passed from one module to the other. The reference numbers for the modules are the same as used in the embodiment illustrated in Fig. 2. The output of the USB interface 208 represents the USB commands. The USB commands are passed on to two receiving entities: one is the input identification module, the other one is the selective command blocker. The output of the input identification module and the decision logic represent internal control commands and information. The latter serves as a switch to let the commands pass or not to pass, based on the input of the decision logic module, which in turn receives information from the input identification module, that is supplied with the same USB command that also the selective USB command blocker receives.

In a further aspect, a computer-readable medium having stored thereon the computer program is provided. The computer-readable medium could be for example a non-transitory computer readable medium. The computer program may be cloud-based data stored in logical pools across disparate, commodity storage servers located on premises or in a data center managed by a third-party cloud provider, such as a third party healthcare provider.

It shall be understood that the system, the method, the computer program, and the computer-readable medium, may have similar and/or corresponding features.

It shall further be understood that one or more of the aforementioned embodiments of the invention may be combined as long as the combined embodiments are not mutually exclusive.

Although various embodiments of the claimed subject matter have been described above with a certain degree of particularity, or with reference to one or more individual embodiments, those skilled in the art could make numerous alterations to the disclosed embodiments without departing from the spirit or scope of the claimed subject matter.

Still other embodiments are contemplated. It is intended that all matter contained in the above description and shown in the accompanying drawings shall be interpreted as illustrative only of particular embodiments and not limiting. Changes in detail or structure may be made without departing from the basic elements of the subject matter as defined in the following claims.

## Claims

1. A method (400) of operating a medical system, comprising:
receiving (402) commands from local and/or remote universal serial bus, USB, input devices through a USB interface, wherein each of the local and remote USB input devices (202, 204) has unique USB input device identifier;
comparing (404) USB input device identifiers with predetermined local USB input device identifiers, thereby identifying whether the commands originate from local or remote USB devices;
determining (406) an action to be pursued for the received commands based on where the commands originate from;
selectively transmitting (408) commands to the medical system based on the determined actions.

2. The method according to claim 1, wherein a locally stored configuration list comprises the predetermined local USB identifiers.

3. The method according to claim 1, further comprising:
requesting a user to interact with a local USB input device;
assigning the local USB input device a local USB input device identifier;
storing the local USB input device identifier as configuration list in a memory.

4. The method according to claim 2 or 3, comprising:
permitting a user to modify the predetermined local USB device identifiers by editing the configuration list.

5. The method according to any of the preceding claims, wherein determining the action to be pursued for the received commands is further based on authorization level associated to the USB input device identifier.

6. The method according to claim 5, wherein the authorization level is comprised in an authorization configuration list stored in a memory and wherein optionally, the authorization configuration list is editable for assigning authorization level to remote USB input device identifiers.

7. The method of any of the preceding claims, wherein selectively transmitting commands to the medical system comprises blocking at least a command received from the remote USB input device.

8. The method of any of the preceding claims, further comprising:
indicating (410) that commands of a remote USB input device are selectively transmitted to the medical system.

9. The method of claim 8, wherein indicating comprises alerting a local user for attempted unauthorized commands from a remote USB input device.

10. The method of claim 8 or 9, wherein indicating comprises remote monitoring of attempted unauthorized commands from a remote USB input device.

11. The method of any of the preceding claims, further comprising:
logging (412) the action for the received commands originating from remote USB input devices.

12. The method of claim 11, wherein logging comprises log-entries of at least one of a timestamp, user action, examination context.

13. A system (100) for operating a medical system, comprising:
a USB interface (108, 208) adapted for connection of local and remote USB input devices;
a computational system (104) configured to perform a method according to any of the claims 1 to 12.

14. The system of claim 13, further comprising:
a display screen;
a magnetic resonance imaging system as medical system.

15. A computer program comprising machine executable instructions, wherein execution of the machine executable instructions causes a computational system to perform a method according to any of the claims 1 to 12.
